# EUROPEAN PATENT APPLICATION

(11) **EP 2 837 619 A1**
(43) Date of publication of application: **18.02.2015**
(21) Application number: 13775610.2
(22) Date of filing: 27.03.2013
(51) Int. Cl.: C07C 231/02, C07C 235/04, C07B 61/00

(54) **METHOD FOR PRODUCING BETA -ALKOXYPROPIONAMIDE**

(30) Priority: 11.04.2012 JP 2012089921
(71) Applicant: Idemitsu Kosan Co., Ltd, Tokyo 100-8321 (JP)
(72) Inventor: FUJIOKA, Toyozo, Chiba 299-0193 (JP)
(74) Representative: Hoffmann Eitle
(86) International application number: PCT/JP2013/002086
(87) International publication number: WO 2013/153754

(57) **Abstract**

A method for producing a β-alkoxypropionamide including reacting a β-alkoxypropionic acid ester represented by the following formula (1) and an amine represented by the following formula (2) in the presence of a polyol and a basic catalyst, thereby to produce a β-alkoxypropionamide represented by the following formula (3),
wherein, as the polyol, a reaction residue that is generated by the reaction and contains a used polyol is used: (wherein in the formulas, R and R' are independently a C₁ to C₆ linear, branched or cyclic, saturated hydrocarbon group; R₁ and R₂ are independently a C₁ to C₆ linear, branched or cyclic, saturated hydrocarbon group; and R₃ is H or CH₃).

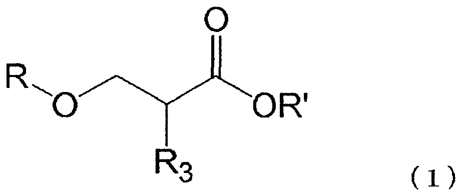

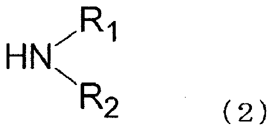

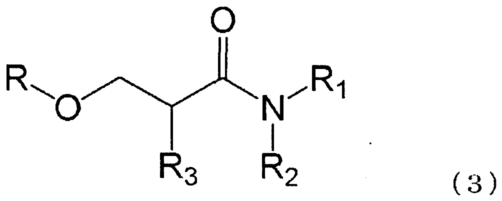

## Description

### Technical Field

The invention relates to a method for producing a β-alkoxypropionamide.

### Background Art

In general, an amide-based organic solvent has an excellent solubility and capability of being dissolved easily in water. Accordingly, this solvent enables rinsing with water, and has desired properties as a solvent or as a washing agent. For example, an amide-based organic solvent can be used as a photoresist peeling agent or a special solvent for a hardly-soluble resin such as polyimide and polyamide.

Generally, N-methylpyrrolidone (NMP) is widely used. However, NMP is suspected for its genetoxicity for a human body, and development of an alternative solvent is earnestly desired. An alternative solvent for NMP has been particularly required for an inkjet ink solvent, an inkjet ink additive, a solvent for producing polyurethane, a solvent for producing polyimide, a polyimide varnish solvent, a paint solvent, an ink solvent, a dispersant, a washing agent, a photoresist peeling agent or the like. A β-alkoxypropionamide, in particular, β-methoxy-N,N-dimethylpropionamide and β-n-butoxy-N,N-dimethylpropionamide or the like are superior to NMP in respect of performance such as boiling point and solubility. Therefore, they are greatly expected in the above-mentioned application fields.

A method for producing a β-alkoxypropionamide has conventionally been known. Patent Document 1 discloses a method in which N,N-dimethylacrylamide or the like are allowed to react with alcohol in the presence of a basic catalyst. By this method, an intended product can be obtained with a high selectivity and with a high yield. This method has a defect that, acrylamide as the raw material is expensive, and N,N-dialkylamide other than N,N-dimethylacrylamide is not easily available on the commercial basis. Therefore, this method is not suited as the industrial method for producing a β-alkoxypropionamide.

Patent Document 2 discloses a method in which a β-alkoxypropionic acid ester is subjected to amidation by an amine in the co-presence of a polyol.

However, selectivity of a β-alkoxypropionamide based on a 3-alkoxypropionic acid ester obtained by this method is surely not high.

Patent Document 3 proposes a method in which a β-alkoxypropionamide is obtained by the reaction of a β-alkoxypropionic acid, a polyol ester and an amine. This method has a defect that it requires a large number of reaction steps. Specifically, it requires 3 steps of reaction, i.e. alkoxylation of an acrylic acid ester, transesterification of a β-alkoxypropionic acid and a polyol and amidation of a transesterified product of a polyol ester, starting from an acrylic acid ester as the raw material of a β-alkoxypropionic acid.

As mentioned above, methods for producing a β-alkoxypropionamide that have been conceived up to date have various problems, and a production method that can solve these problems has been required.

### Related Art Documents

### Patent Documents

Patent Document 1: JP-A-2004-250353
Patent Document 2: WO2008/102615
Patent Document 3: JP-A-2010-138094

### Summary of the Invention

An object of the invention is to provide a method for producing a β-alkoxypropionamide in a high selectivity and high yield.

The inventors made intensive studies. As a result, the inventors have found that, in a reaction in which a β-alkoxypropionic acid ester is subjected to amidation in the co-presence of a polyol and a basic catalyst, by neutralizing the basic catalyst after the reaction and re-using without disposing and reacting a reaction residue obtained by distillation of a formed product instead of the polyol, the selectivity for a β-alkoxypropionic acid ester is increased, and a formed product can be obtained with a high yield. The invention has been attained based on this finding.

According to the invention, the following method for producing a β-alkoxypropionamide is provided.
1. A method for producing a β-alkoxypropionamide comprising reacting a β-alkoxypropionic acid ester represented by the following formula (1) and an amine represented by the following formula (2) in the presence of a polyol and a basic catalyst, thereby to produce a β-alkoxypropionamide represented by the following formula (3),
   wherein, as the polyol, a reaction residue that is generated by the reaction and contains a used polyol is used: 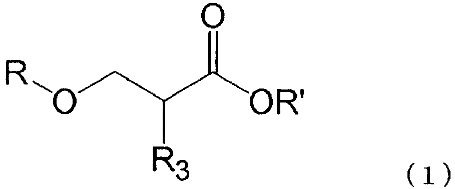 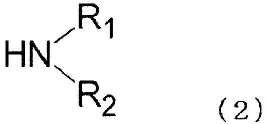 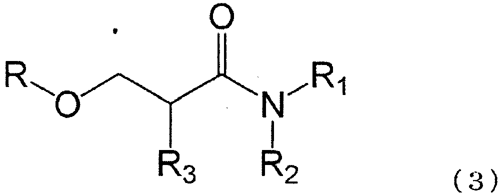 wherein in the formulas, R and R' are independently a C₁ to C₆ linear, branched or cyclic, saturated hydrocarbon group; R₁ and R₂ are independently a C₁ to C₆ linear, branched or cyclic, saturated hydrocarbon group; and R₃ is H or CH₃.
2. The method according to 1, wherein the polyol is glycerin, diglycerin or diethylene glycol.
3. The method according to 1 or 2, wherein the basic catalyst is NaOR₄ (wherein R₄ is a C₁ to C₆ linear or branched, saturated hydrocarbon group) or potassium t-butoxide.
4. The method according to any of 1 to 3, wherein R in the formulas (1) and (3) is methyl or n- butyl.
5. The method according to any of 1 to 4, wherein R₁ and R₂ in the formulas (2) and (3) are methyl.

According to the invention, it is possible to provide a method for producing a β-alkoxypropionamide with a high selectivity and yield.

### Mode for Carrying out the Invention

The method for producing a β-alkoxypropionamide according to the invention is characterized in that, in a method for producing a β-alkoxypropionamide by reacting a β-alkoxypropionic acid ester represented by the following formula (1) with an amine represented by the following formula (2) in the presence of a polyol and a basic catalyst, thereby to produce a β-alkoxypropionamide represented by the following formula (3), as the polyol, a reaction residue that is generated by the reaction and contains the used polyol is utilized. 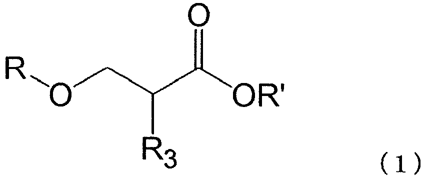 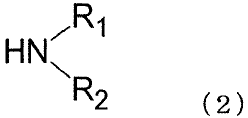 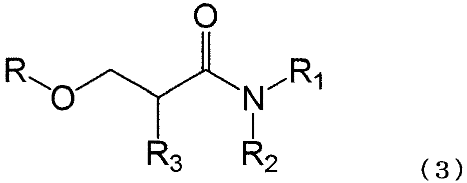 wherein in the formula, R and R' are independently a C₁ to C₆ linear, branched or cyclic saturated hydrocarbon group; R₁ and R₂ are independently a C₁ to C₆ linear, branched or cyclic, saturated hydrocarbon group; and R₃ is H or CH₃.

The reaction residue to be used can be obtained by neutralizing the reaction liquid and distilling off a β-alkoxypropionamide as a formed product, a by-product or the like.

At this time, a neutralized salt of the basic catalyst can be separated by an operation such as filtration before, during or after the distillation of the formed product.

In the production method of the invention, recovery and re-use of a reaction residue containing a polyol can be conducted at least 5 times or more. Afresh polyol is added only at the time of the initial reaction. Thereafter, it may be added in only a small amount such that the total amount becomes an adequate amount, and there is no need to add it in a large amount.

In conventional methods, a fresh polyol is used for each reaction and the used alcohol is then disposed. In contrast, the production method of the invention is an industrial method that can reduce the amount of a waste disposed in order to protect global environment, and at the same time, can attain economic advantages.

As for the β-alkoxypropionic acid ester represented by the formula (1) that is used in the invention, in the formula, R and R' are independently a C₁ to C₆ (preferably C₁ to C₄) linear, branched or cyclic, saturated hydrocarbon group, and R₃ is H or CH₃.

The β-alkoxypropionic acid ester represented by the formula (1) can be produced by a method that is generally known. For example, it can be obtained by reacting an ester such as methyl acrylate or methyl methacrylate with a corresponding alcohol in the presence of a basic catalyst.

The R group of the alcohol (ROH) used at this time is a C₁ to C₆ linear or branched hydrocarbon group. Specific examples of alcohol include, for example, methanol, ethanol, n-propanol, isopropanol, n-butanol, sec-butanol, iso-butanol, n-pentanol, cyclopentanol, isopentanol, n-hexanol, isohexanol and cyclohexanol.

As specific examples of the β-alkoxypropionic acid ester represented by the formula (1), methyl 3-methoxypropionate, ethyl 3-ethoxypropionate, n-propyl 3-n-propoxy propionate, isopropy 3-isopropoxy propionate, n-butyl 3-n-butoxy propionate, sec-butyl 3-sec-butoxypropionate, isobutyl 3-isobutoxypropionate, n-pentyl 3-n-pentyloxy propionate, cyclopentyl 3-cyclopentyloxypropionate, isopentyl 3-isopentyloxypropionate, n-hexyl 3-n-hexaoxypropionate, isohexyl 3-isohexaoxypropionate, cyclohexyl 3-cyclohexaoxypropionate, methyl 3-methoxy-2-methylpropionate, ethyl 3-ethoxy-2-methylpropionate, n-propyl 3-n-propoxy-2-methoxy propionate, isopropyl 3-isopropoxy 2-methylpropionate, n-butyl 3-n-butoxy-2-methylpropionate, sec-butyl 3-sec-butoxy-2-methylpropionate, isobutyl 3-isobutoxy 2-methylpropionate, n-pentyl 3-n-pentyloxy-2-methylpropionate, cyclopentyl 3-cyclopentyloxy-2-methylpropionate, isopentyl 3-isopentyloxy-2-methylpropionate, n-hexyl 3-n-hexaoxy-2-methylpropionate, isohexyl 3-isohexaoxy-2-methylpropionate, cyclohexyl 3-cyclohexaoxy-2-methylpropionate, or the like can be given, for example.

It is preferred that R and R' for the β-alkoxypropionic acid ester represented by the formula (1) be independently methyl or n-butyl.

The amines represented by the formula (2) and used in the method of the invention are secondary amines. In the formula, R₁ and R₂ are independently a C₁ to C₆ (preferably C₁ to C₄) linear, branched or cyclic saturated hydrocarbon group.

As specific examples of the amines represented by the formula (2), dimethylamine, diethylamine, methylethylamine, di-n-propylamine, n-propyl methylamine, n-propyl ethylamine, di-sec-n-propylamine, sec-propyl methylamine, sec-propyl ethylamine, di-n-butylamine, n-butyl methylamine, n-butyl ethylamine, n-butyl n-propylamine, n-butyl isopropylamine, di-sec-butylamine, sec-butyl methylamine, sec-butyl ethylamine, sec-butyl n-propylamine, sec-butyl isopropylamine, dipentylamine, dihexylamine, piperidine, pyrrole or the like can be given.

It is preferred that R₁ and R₂ in the amines represented by the formula (2) are methyl. That is, dimethylamine is preferable.

As for the β-alkoxypropionamide produced by the method of the invention, the boiling point thereof is required to be low to a certain degree in order to be purified by distillation. In this regard, R₁ and R₂ are independently a C₁ to C₆ (preferably C₁ to C₄) linear, branched or cyclic, saturated hydrocarbon group. Preferably, R₁ and R₂ are methyl.

As for the β-alkoxypropionamide represented by the following formula (3) that is produced by the method of the invention, in the formula, R is a C₁ to C₆ (preferably C₁ to C₄) linear, branched or cyclic saturated hydrocarbon group. R₁ and R₂ are independently a C₁ to C₆ (preferably C₁ to C₄) linear, branched or cyclic saturated hydrocarbon group.

In the β-alkoxypropionamide represented by the following formula (3), the total number of carbon atoms of R, R₁, R₂ and R₃ is preferably 3 to 8, more preferably 3 to 6. If the total number of carbon atoms exceeds 8, the boiling point of a formed product becomes high, resulting in difficulty in removal by distillation of the formed product after neutralizing of the reaction liquid.

As specific examples of the β-alkoxypropionamide represented by the formula (3) that is produced by the method of the invention, the following can be given, for example.
3-methoxy-N,N-dimethylpropionic acid amide, 3-ethoxy-N,N-dimethylpropionic acid amide, 3-n-propoxy-N,N-dimethylpropionic acid amide,
3-isopropoxy-N,N-dimethylpropionic acid amide, 3-n-butoxy-N,N-dimethylpropionic acid amide, 3-sec-butoxy-N,N-dimethylpropionic acid amide,
3-isobutoxy-N,N-dimethylpropionic acid amide, 3-pentyloxy-N,N-dimethylpropionic acid amide, 3-hexyloxy-N,N-dimethylpropionic acid amide, 3-methoxy-N,N-diethylpropionic acid amide, 3-ethoxy-N,N-diethylpropionic acid amide, 3-n-propoxy-N,N-diethylpropionic acid amide, 3-isopropoxy N,N-diethylpropionic acid amide,
3-n-butoxy-N,N-diethylpropionic acid amide, 3-sec-butoxy-N,N-diethylpropionic acid amide, and 3-isobutoxy-N,N-diethylpropionic acid amide.

3-methoxy-N,N-methylethylpropionic acid amide,
3-ethoxy-N,N-methylethylpropionic acid amide, 3-n-propoxy-N,N-methylethylpropionic acid amide, 3-isopropoxy-N,N-methylethylpropionic acid amide,
3-n-butoxy-N,N-methylethylpropionic acid amide,
3-sec-butoxy-N,N-methylethylpropionic acid amide,
3-isobutoxy-N,N-methylethylpropionic acid amide, 3-pentyloxy-N,N-methylethylpropionic acid amide, 3-methoxy-N,N-di-n-propylpropionic acid amide,
3-ethoxy-N,N-di-n-propylpropionic acid amide, 3-methoxy-N,N-diisopropylpropionic acid amide, and 3-ethoxy-N,N-diisopropylpropionic acid amide.

3-methoxy-N,N-methyl-n-propylpropionic acid amide,
3-ethoxy-N,N-methyl-n-propylpropionic acid amide,
3-n-propoxy-N,N-methyl-n-propylpropionic acid amide,
3-isopropoxy-N,N-methyl-n-propylpropionic acid amide,
3-n-butoxy-N,N-methyl-n-propylpropionic acid amide,
3-sec-butoxy-N,N-methyl-n-propylpropionic acid amide,
3-isobutoxy-N,N-methyl-n-propylpropionic acid amide,
3-methoxy-N,N-methylisopropylpropionic acid amide,
3-ethoxy-N,N-methylisopropylpropionic acid amide,
3-n-propoxy-N,N-methylisopropylpropionic acid amide,
3-isopropoxy-N,N-methylisopropylpropionic acid amide,
3-n-butoxy-N,N-methylisopropylpropionic acid amide,
3-sec-butoxy-N,N-methylisopropylpropionic acid amide, and
3-isobutoxy-N,N-methylisopropylpropionic acid amide.

3-methoxy-N,N-ethylisopropylpropionic acid amide,
3-ethoxy-N,N-ethylisopropylpropionic acid amide,
3-n-propoxy-N,N-ethylisopropylpropionic acid amide,
3-isopropoxy-N,N-ethylisopropylpropionic acid amide,
3-methoxy-N,N-ethyl-n-propylpropionic acid amide,
3-ethoxy-N,N-ethyl-n-propylpropionic acid amide,
3-n-propoxy-N,N-ethyl-n-propylpropionic acid amide, and
3-isopropoxy-N,N-ethyl-n-propylpropionic acid amide.

3-methoxy-2-methyl-N,N-dimethylpropionic acid amide,
3-ethoxy-2-methyl-N,N-dimethylpropionic acid amide,
3-n-propoxy-2-methyl-N,N-dimethylpropionic acid amide,
3-isopropoxy-2-methyl-N,N-dimethylpropionic acid amide,
3-n-butoxy-2-methyl-N,N-dimethylpropionic acid amide,
3-sec-butoxy-2-methyl-N,N-dimethylpropionic acid amide,
3-isobutoxy-2-methyl-N,N-dimethylpropionionic acid amide,
3-pentyloxy-2-methyl-N,N-dimethylpropionic acid amide,
3-methoxy-2-methyl-N,N-diethylpropionic acid amide,
3-ethoxy-2-methyl-N,N-diethylpropionic acid amide,
3-n-propoxy-2-methyl-N,N-diethylpropionic acid amide, and
3-isopropoxy-2-methyl-N,N-diethylpropionic acid amide.

3-methoxy-2-methyl-N,N-methylethylpropionic acid amide,
3-ethoxy-2-methyl-N,N-methylethylpropionic acid amide,
3-n-propoxy-2-methyl-N,N-methylethylpropionic acid amide,
3-isopropoxy-2-methyl-N,N-methylethylpropionic acid amide,
3-n-butoxy-2-methyl-N,N-methylethylpropionic acid amide,
3-sec-butoxy-2-methyl-N,N-methylethylpropionic acid amide,
3-isobutoxy-2-methyl-N,N-methylethylpropionic acid amide,
3-methoxy-2-methyl-N,N-di-n-propylpropionic acid amide, and

3-methoxy-2-methyl-N,N-diisopropylpropionic acid amide.

3-methoxy-2-methyl-N, N-methyl-n-propylpropionic acid amide,
3-ethoxy-2-methyl-N,N-methyl-n-propylpropionic acid amide,
3-n-propoxy-2-methyl-N,N-methyl-n-propylpropionic acid amide,
3-isopropoxy-2-methyl-N,N-methyl-n-propylpropionic acid amide,
3-methoxy-2-methyl-N,N-methylisopropylpropionic acid amide,
3-ethoxy-2-methyl-N,N-methylisopropylpropionic acid amide,
3-n-propoxy-2-methyl-N,N-methylisopropylpropionic acid amide,
3-isopropoxy-2-methyl-N,N-methylisopropylpropionic acid amide,
3-methoxy-2-methyl-N,N-ethylisopropylpropionic acid amide,
3-ethoxy-2-methyl-N,N-ethylisopropylpropionic acid amide,
3-methoxy-2-methyl-N,N-ethyl-n-propylpropionic acid amide, and
3-ethoxy-2-methyl-N,N-ethyl-n-propylpropionic acid amide.

As for the β-alkoxypropionamide represented by the formula (3), one in which R is methyl or n-butyl is preferable.

As for the β-alkoxypropionamide represented by the formula (3), one in which R₁ and R₂ are methyl is preferable.

As for the β-alkoxypropionamide represented by the formula (3), one in which R₃ is hydrogen is preferable.

More preferably, the β-alkoxypropionamide represented by the formula (3) is 3-methoxy-N,N-dimethylpropionic acid amide or 3-n-butoxy-N,N-dimethylpropionic acid amide.

The method for producing a β-alkoxypropionamide of the invention is particularly preferable as the method for producing 3-methoxy-N,N-dimethylpropionic acid amide or 3-n-butoxy-N,N-dimethylpropionic acid amide.

Since 3-methoxy-N,N-dimethylpropionic acid amide has almost the same boiling point as that of NMP, and solubility for various solutes are very close to that of NMP, it is expected to be used as an alternative solvent for NMP. In addition, 3-n-butoxy-N,N-dimethylpropionic acid amide is amphiphilic, i.e. has properties of being mixed homogeneously with water and oil. Therefore, the use thereof as a compatibilizer and a washing agent is expected in the field of a paint, ink, various varnishes as polyimide varnish, washing of an apparatus for molding polyurethane or the like, dispersion of fine particles (carbon nanotube, carbon powder, abrasive or the like) or the like.

As the polyol, a compound having two or more OH groups within a molecule may be used. Specific examples thereof include glycerin, diglycerin, ethylene glycol, propylene glycol, diethylene glycol, dipropylene glycol, or the like can be used. They may be used singly or in a mixture of two or more. Among them, glycerin, diglycerin or diethylene glycol are preferable.

The basic catalyst may be a common basic catalyst, and the examples thereof include NaOR₄ (R₄ is a C₁ to C₆ linear or branched hydrocarbon group), potassium t-butoxide (t-BuOK), butyl lithium or the like. As for the NaOR₄, specific examples thereof include sodium methoxide, sodium ethoxide, sodium propoxide, butyl lithium or the like. In particular, sodium methoxide or potassium t-butoxide that is easily available on the industrial basis is preferable.

No specific restrictions are imposed on the amount of the basic catalyst. However, if the amount of the basic catalyst is small, the reaction proceeds slowly and takes time. Therefore, in order to allow the reaction to complete within about 6 hours, the amount is preferably 0.005 to 0.05 equivalent relative to the amount of the β-alkoxypropionic acid ester.

At the time of the initial reaction, that is, when a reaction is conducted by using a fresh raw material without using a reaction residue, if the molar ratio of an amine is large, the amount of a by-product tends to be increased. In addition, if the molar ratio of the polyol is large, the selectivity tends to be low. Taking the above into consideration, the amount ratio of the raw material charged is preferably β-alkoxypropionic acid ester (formula 1)/amine (formula 2)/polyol = 1/1 to 3/0.5 to 3 (mole), more preferably β-alkoxypropionic acid ester (formula 1)/amine (formula 2)/polyol = 1/1 to 2/0.5 to 1.5 (mole).

If the reaction temperature is too low, the reaction proceeds slowly. If the reaction temperature is too high, the amount of a by-product tends to be increased. Therefore, the reaction temperature is preferably 0°C to 100°C, more preferably 20°C to 80°C. If the reaction is conducted under such conditions, the reaction is completed within 2 to 6 hours.

For a post treatment after the reaction, by neutralizing a basic catalyst with an appropriate acid (e.g. sulfuric acid, phosphoric acid), removing precipitated neutralized acids by filtration, and removing by distillation of the filtrate, an excessively-added amine, an alcohol as a by-product and a β-alkoxypropionamide as a formed product are distilled off to obtain a reaction residue.

Distillation of the basic catalyst without neutralization is not preferable, since the formed product is decomposed. However, if the amine is dimethylamine and the alcohol that is formed as a by-product is methanol, since distillation can be conducted at a relatively low temperature, the basic catalyst may be neutralized after distillation of them, and the formed product may be distilled thereafter.

At this time, the amount of an acid used for neutralization is preferably 1.00 to 2.0 equivalents, more preferably 1.0 to 1.2 equivalents, relative to the amount of the catalyst added during the reaction.

As for the bottom temperature when removing by distillation of the amine that is excessively added, the alcohol that is produced as a by-product and a formed product, if it exceeds 180°C, part of the formed product is decomposed. Therefore, the temperature at the time of distillation is required to be adjusted by the degree of decompression such that it becomes 180°C or less, more preferably 150°C or less.

The conditions at the time of distillation can be appropriately selected by a person skilled in the art taking into consideration the formed product and the by-product.

The reaction residue thus obtained is not disposed, and used instead of a fresh polyol at the subsequent amidation reaction.

The reaction conditions at the time of using reaction residues (the amount ratio of a β-alkoxypropionic acid ester (formula 1)/an amine (formula 2), the amount of a catalyst, the reaction temperature, the reaction time or the like) are the same as those when a fresh polyalcohol is used at the initial reaction.

However, when an acid that has been added in an excessive amount is present at the time of the previous neutralizing of the catalyst remains, the amount of the catalyst is increased in an amount required for neutralizing it such that the effective catalyst amount is adjusted.

Specifically, after the reaction, if 1.05 equivalents of an acid is added to the added catalyst after the reaction in order to neutralize the basic catalyst, an acid that is equal to 0.05 equivalent is present in a reaction residue in advance. Therefore, it is needed to increase the amount of the catalyst (0.05 equivalent) that is enough to neutralize it.

Further, after conducting a reaction using a reaction residue instead of a fresh polyol, a similar post treatment is conducted, and an amidation reaction using the obtained reaction residue can be conducted repeatedly. This number of possible repetition is found to be 5 times or more.

As mentioned above, in the amidation reaction using the resulting reaction residue, as compared with the case where a fresh polyol is used, the selectivity based on a β-alkoxypropionic acid ester is increased. At the same time, the amount of a polyol used can be significantly decreased. Further, the amount of a waste can be reduced.

### EXAMPLES

### Production Example 1 Synthesis of β-alkoxypropionic acid ester

By using the method disclosed in WO2008/102615, methyl 3-methoxypropionate and n- butyl 3-n-buthoxypropionate were synthesized. They were used as the raw materials in the following Comparative Examples and Examples.

### Comparative Example 1

Methyl 3-methoxypropionate, sodium methoxide (MeONa) shown in Table 1 and glycerin (polyol) were placed in a 300 mL-autoclave. A reaction was conducted at 60°C for 6 hours. After cooling, the autoclave was depressurized, and the content was taken out. A small amount of the content was taken out, and by the internal standard method of gas chromatography (internal standard material: NMP), methyl 3-methoxypropionate as the raw material, 3-methoxy-N,N-dimethylpropionic amide as the formed product were quantified. From the results of quantification, by calculation, the conversion of methyl 3-methoxypropionic acid and the selectivity and yield based on methyl 3-methoxypropionate were obtained. The results are shown in Table 1.

The content of the autoclave after the reaction was transferred to a 300 mL-beaker. 98% concentrated sulfuric acid in an amount shown in Table 1 was added while stirring by a glass bar, whereby sodium methoxide as the catalyst was neutralized and allowed to be precipitated as white salts. Thereafter, white salts were removed by suction filtration, and the filtrate was then concentrated by means of an evaporator. At this time, for heating, an oil bath that was incubated at 100°C was used, and the degree of decompression was gradually increased with care so that the content could be prevented from bumping. When the degree of decompression reached 2 mmHg, the concentration operation was completed, whereby a yellow brown reaction residue in an amount shown in Table 1 were obtained. The distillate was confirmed to be the amine that had excessively been added, the alcohol as the by-product and the intended product by a GC (gas chromatography) analysis.

### Examples 1 to 5

A reaction was conducted in the same manner as in Comparative Example 1, except that the reaction residue shown in Table 1 was used instead of glycerin, and the conversion ratio, the selectivity and the yield were calculated. A reaction residue was obtained in the same manner as in Comparative Example 1.

That is, in Example 1, the reaction residue obtained in Comparative Example 1 was used. In each of Examples 2 to 5, a reaction residue obtained in the previous example was used.

The results are shown in Table 1.

### Comparative Example 2

A reaction was conducted in the same manner as in Comparative Example 1, except that n- butyl 3-n-butoxypropionate was used instead of methyl 3-methoxypropionate, potassium t-butoxide (t-BuOK) was used instead of sodium methoxide and diglycerin (polyol) was used instead of glycerin, whereby the conversion ratio of n-butyl 3-n-butoxypropionate as the raw material, the selectivity based on n- butyl 3-n-buthoxypropionate and the yield of 3-n-butoxy-N,N-dimethylpropionamide as the formed product were obtained. The results are shown in Table 2.

Further, in the same manner as in Comparative Example 1, potassium t-butoxide as the catalyst was neutralized and removed, and the residue was concentrated, whereby the reaction residue in the amount shown in Table 2 was obtained.

### Examples 7 to 10

Reactions were conducted in the same manner as in Comparative Example 2, except that the reaction residue shown in Table 2 was used instead of diglycerin, and the conversion ratio, the selectivity and the yield were calculated. The reaction residue was obtained in the same manner as in Comparative Example 2.

That is, in Example 7, the reaction residue obtained in Comparative Example 2 was used. In each of Examples 8 to 10, a reaction residue obtained in the previous example was used.

The results are shown in Table 2.

**Table 1**

| | | Comp. Ex. 1 | Ex. 1 | Ex. 2 | Ex. 3 | Ex. 4 | Ex. 5 |
|---|---|---|---|---|---|---|---|
| Compounding of raw materials | Methyl 3-methoxypropionate (mol) | 0.6 | 0.6 | 0.6 | 0.6 | 0.6 | 0.6 |
| | MeONa (mol) | 0.03 | 0.032 | 0.032 | 0.032 | 0.032 | 0.032 |
| | Glycerin (mol) | 0.6 | 0 | 0 | 0 | 0 | 0 |
| | Reaction residue used | - | Comp. Ex. 1 | Ex. 1 | Ex. 2 | Ex. 3 | Ex. 4 |
| | Reaction residue (g) | 0 | 66.3 | 64.2 | 63.8 | 62.8 | 63.1 |
| | Dimethylamine (mol) | 0.9 | 0.9 | 0.9 | 0.9 | 0.9 | 0.9 |
| Reaction conditions | Temperature (°C) | 60 | 60 | 60 | 60 | 60 | 60 |
| | Hour (h) | 6 | 6 | 6 | 6 | 6 | 6 |
| Amount of acid for neutralization | Sulfuric acid (mol) | 0.016 | 0.017 | 0.017 | 0.017 | 0.017 | 0.017 |
| Results | Conversion ratio of methyl 3-methoxypropionate (%) | 99 | 99 | 98 | 97 | 98 | 97 |
| | Selectivity based on methyl 3-methoxypropionate (%) | 80 | 90 | 86 | 87 | 86 | 88 |
| | Yield (%) | 79 | 89 | 85 | 86 | 85 | 87 |
| | Reaction residue (g) | 66.3 | 64.2 | 63.8 | 62.8 | 63.1 | 62.5 |

**Table 2**

| | | Comp. Ex. 2 | Ex. 6 | Ex. 7 | Ex. 8 | Ex. 9 | Ex. 10 |
|---|---|---|---|---|---|---|---|
| Compounding of raw materials | N- butyl 3-n-buthoxypropionate (mol) | 0.6 | 0.6 | 0.6 | 0.6 | 0.6 | 0.6 |
| | t-BuOK (mol) | 0.03 | 0.032 | 0.032 | 0.032 | 0.032 | 0.032 |
| | Diglycerin (mol) | 0.6 | 0 | 0 | 0 | 0 | 0 |
| | Reaction residue used | - | Comp. Ex. 2 | Ex. 6 | Ex. 7 | Ex. 8 | Ex. 9 |
| | Reaction residue (g) | 0 | 119.6 | 100.3 | 99.5 | 100.8 | 99.2 |
| | Dimethylamine (mol) | 0.9 | 0.9 | 0.9 | 0.9 | 0.9 | 0.9 |
| Reaction conditions | Temperature (°C) | 60 | 60 | 60 | 60 | 60 | 60 |
| | Time (h) | 6 | 6 | 6 | 6 | 6 | 6 |
| Amount of acid for neutralization | Sulfuric acid (mol) | 0.016 | 0.017 | 0.017 | 0.017 | 0.017 | 0.017 |
| Results | Conversion ratio of n- butyl 3-n-buthoxypropionate (%) | 99 | 99 | 98 | 97 | 98 | 97 |
| | Selectivity based on n- butyl 3-n-butoxypropionate (%) | 77 | 85 | 82 | 86 | 81 | 85 |
| | Yield (%) | 76 | 84 | 81 | 85 | 80 | 84 |
| | Reaction residue (g) | 119.6 | 100.3 | 99.5 | 100.8 | 99.2 | 99.3 |

From Table 1 and Table 2, it has been revealed that, by using the reaction residues instead of the polyol, the selectivity is increased while maintaining almost the same conversion ratio, and as a result, the yield of 3-alkoxy-N,N-dimethylpropionic acid amide as an intended product is improved.

In addition, the reaction residue can be used as it is without conducting a purification treatment such as purification by distillation. Further, it can be re-used five times or more.

According to the method of the invention, it is possible to produce a β-alkoxypropionamide with a high selectivity and with a high yield. In addition, since there is no need to dispose a reaction residue, the method of the invention is advantageous in respect of global environment and economy.

### Industrial Applicability

According to the method of the invention, a β-alkoxypropionamide can be produced with a high selectivity and with a high yield. Therefore, the method of the invention is significantly effective as the method for producing a β-alkoxypropionamide. A β-alkoxypropionamide produced by this method is effective as a solvent.

Although only some exemplary embodiments and/or examples of this invention have been described in detail above, those skilled in the art will readily appreciate that many modifications are possible in the exemplary embodiments and/or examples without materially departing from the novel teachings and advantages of this invention. Accordingly, all such modifications are intended to be included within the scope of this invention.

The documents described in this specification and the Japanese application specification claiming priority under the Paris Convention are incorporated herein by reference in its entirety.

## Claims

1. A method for producing a β-alkoxypropionamide comprising reacting a β-alkoxypropionic acid ester represented by the following formula (1) and an amine represented by the following formula (2) in the presence of a polyol and a basic catalyst, thereby to produce a β-alkoxypropionamide represented by the following formula (3),
wherein, as the polyol, a reaction residue that is generated by the reaction and contains a used polyol is used: 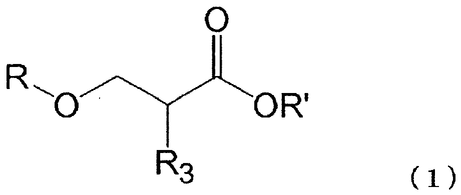 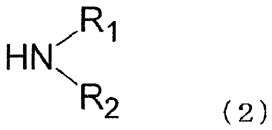 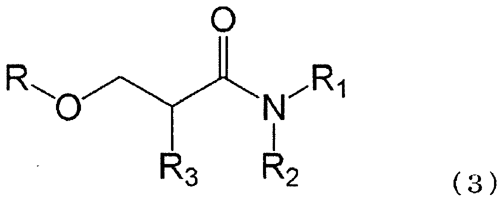 wherein in the formulas, R and R' are independently a C₁ to C₆ linear, branched or cyclic, saturated hydrocarbon group; R₁ and R₂ are independently a C₁ to C₆ linear, branched or cyclic, saturated hydrocarbon group; and R₃ is H or CH₃.

2. The method according to claim 1, wherein the polyol is glycerin, diglycerin or diethylene glycol.

3. The method according to claim 1 or 2, wherein the basic catalyst is NaOR₄ (wherein R₄ is a C₁ to C₆ linear or branched, saturated hydrocarbon group) or potassium t-butoxide.

4. The method according to any of claims 1 to 3, wherein R in the formulas (1) and (3) is methyl or n-butyl.

5. The method according to any of claims 1 to 4, wherein R₁ and R₂ in the formulas (2) and (3) are methyl.
